# EUROPEAN PATENT SPECIFICATION

(11) **EP 2 266 438 B1**
(45) Date of publication and mention of the grant of the patent: **06.01.2016**
(21) Application number: 09732820.7
(22) Date of filing: 07.04.2009
(51) Int. Cl.: A47C 3/02, A47C 3/025, A61H 23/02, A61M 21/00, A61M 21/02

(54) **RELAXATION DEVICE**
ENTSPANNUNGSVORRICHTUNG
DISPOSITIF DE RELAXATION

(30) Priority: 17.04.2008 JP 2008108002
(43) Date of publication of application: 29.12.2010
(73) Proprietor: Panasonic Corporation, Osaka 571-8501 (JP)
(72) Inventor: MORIKAWA, Daisuke, Osaka-shi Osaka 450-6207 (JP); MICHIMORI, Akihiro, Osaka-shi Osaka 540-6207 (JP)
(74) Representative: Appelt, Christian W.
(86) International application number: PCT/JP2009/057094
(87) International publication number: WO 2009/128362

(56) References cited:
- JP-A- 11 137 626
- JP-A- 2001 149 164
- JP-A- 2001 190 677
- JP-A- 2007 037 832
- US-A- 6 155 976

## Description

### TECHNICAL FIELD

The present invention relates to a relaxation device that has a relaxing and refreshing effects on a user.

### BACKGROUND ART

Document JP 2000-42112 discloses a prior art example of a relaxation device that rocks the body of a user who is seated on a chair-shaped body support and has a relaxing effect on the user.

The relaxation device of document JP 2000-42112 includes a rocking driver (vibration means in document JP 2000-42112) that rocks the body support in forward and rearward directions. The rocking driver rocks the body support at a frequency of 1 Hz or less and gradually varies the frequency with a variation width of 0.2 Hz or less.

Document US 6155976 A discloses a reciprocating platform assembly for oscillating a subject supporting platform in a back and forth, headward to footward motion includes a platform mounted on displacement modules that are mounted to a frame. The displacement modules are signally connected to a processor that controls movement of the platform through operation of the displacement modules.

### DISCLOSURE OF THE INVENTION

In the above-described relaxation apparatus, the rocking of the user's body has a relaxing effect on the user. This relaxes the body and may result in, for example, the user falling asleep.

The present invention provides a relaxation device that rocks the user's body so that it first has a relaxing effect on the user and then has a refreshing effect on the user.

To achieve the above object, a relaxation device includes a body supporting means for supporting a user's body, a rocking driving means for rocking the body supporting means, and a control means for controlling the rocking driving means. The control means controls the rocking driving means to perform relaxation rocking that rocks the body supporting means at a frequency in a range of 1 Hz or less and fluctuates at least either one of the frequency and amplitude of the rocking. Further, the control means includes a refreshment control means that controls the rocking driving means to perform, subsequent to the relaxation rocking, refreshment rocking that stops the fluctuation and increases at least either one of the rocking frequency and amplitude of the body supporting means thereby refreshing the user who has been in a relaxed state.

In this invention, the control means includes the refreshment control means that controls the rocking driving means to perform, subsequent to the relaxation rocking, refreshment rocking that stops the fluctuation and increases at least either one of the rocking frequency and amplitude of the body supporting means thereby refreshing the user who has been in a relaxed state. This increases at least either one of the rocking per unit time and the rocking width during the refreshment operation that increases either one of the rocking frequency and amplitude and has a refreshing effect on the user, who has been in a relaxed state due to the relaxation rocking, thereby drawing the user from the relaxed state to an awake state.

In one aspect, the refreshment control means controls the rocking driving means so as to increase both of the rocking frequency and amplitude. In comparison to when increasing only either one of the frequency and amplitude, this increases both of the rocking frequency and amplitude thereby increasing the rocking per unit time and the rocking width. Thus, the refreshing effect that effectively draws the user from a relaxed state into an awake state is further effective.

In one aspect, the refreshment control means controls the rocking driving means so as to synchronously increase both of the rocking frequency and amplitude. Since the rocking per unit time and the rocking width are simultaneously increased, the refreshing effect that effectively draws the user from a relaxed state into an awake state is further effective.

In one aspect, the control means controls the rocking driving means so as to gradually decrease the frequency and fluctuate the amplitude during the relaxation rocking. This decreases the rocking frequency so as to assist the user's breathing and fluctuates the rocking amplitude thereby producing a relaxing effect that draws the user from an awake state to a relaxed state.

In one aspect, the control means varies the frequency of the relaxation rocking in a range of 0.2 Hz to 0.4 Hz, and the range of at least either one of the frequency and amplitude of the refreshment rocking performed by the refreshment control means is greater than that of the relaxation rocking. In other words, during the relaxation rocking, the frequency is varied within the range of 0.2 Hz to 0.4 Hz, which is close to the breathing frequency when the user (person) is relaxed, and thus draws the user into a relaxed state. Further, since at least either one of the frequency and amplitude of the refreshment rocking performed by the refreshment control means is greater than that of the relaxation rocking, the relaxing effect that draws the user into a relaxed state is further effective.

In one aspect, the frequency of the refreshment rocking performed by the refreshment control means is varied at a rate that is greater than the rate at which the frequency of the relaxation rocking is varied. Since the varying rate of the frequency is greater in the refreshment rocking than the relaxation rocking, the refreshment rocking provides the user with a relatively strong stimulus and has a further effective refreshing effect on the user.

According to the invention, the body support means is formed by a backrest fixed in an inclinable manner to a rear part of a seat on which the user seats. Further, the control means inclines the backrest to a reclined state during the relaxation rocking, and the refreshment control means raises the backrest from the reclined state. By raising the backrest upright, the user's upper body is also raised. This has a further effective refreshing effect on the user. Further, the user's body may be held in a state close to a state in which the user is lying down. This provides a relaxing effect.

In one aspect, the refreshment control means raises the backrest in a stepped manner. This raises the user's upper body in a stepped manner. Thus, sudden changes in the user's blood pressure or the like are suppressed, and the burden on the user is suppressed.

In one aspect, the control means inclines the backrest in a stepped manner to the reclined state during the relaxation rocking. This lowers the user's upper body in a stepped manner. Thus, sudden changes in the user's blood pressure or the like are suppressed, and the burden on the user is suppressed.

In one aspect, the control means inclines the backrest synchronously with the varying of either one of frequency and amplitude. This synchronizes (harmonizes) the backrest with either one of frequency and amplitude and has a further effective relaxing and refreshing effects on the user.

In one aspect, the refreshment control means inclines the backrest so that the speed at which the backrest moves is higher during the refreshment rocking than the speed at which the backrest moves during the relaxation rocking. The speed at which the backrest moves during the refreshment rocking is higher than that during the relaxation rocking. This has a further

According to the invention, at least either one of the seat and the backrest includes pairs of left and right airbags that are inflated and deflated when air from an air pump is supplied and released, and the refreshment control means controls the air pump so as to inflate and deflate the left airbag and the right airbag with a time difference during the refreshment rocking to turn the user. This turns, or stretches, the user's right body half (upper right body half) and left body half (upper left body half) and has a further effective refreshing effect on the user.

In one aspect, the backrest includes a back airbag that presses the user's back by supplying and releasing air from an air pump, and the refreshment control means controls the air pump so as to inflate and deflate the back airbag during the refreshment rocking. This mainly stretches the user's back and has a further effective refreshing effect on the user.

In one aspect, the control means also controls the air pump to inflate and deflate the airbags during the relaxation rocking. Further, the control means keeps the rocking frequency at a lower limit value, fluctuates the amplitude, and stops inflating and deflating the airbags when sleep rocking is performed during the relaxation rocking. During sleep rocking, the generation of operational noise from the air pump or the like related with the inflation and deflation of the airbags that may interfere with the user's sleep is suppressed, and the effect of the sleep rocking is further increased.

### BRIEF DESCRIPTION OF THE DRAWINGS

Fig. 1 is a schematic diagram of a relaxation device according to one embodiment;
Fig. 2 is a perspective view showing a body support;
Fig. 3 is a timing chart showing the rocking amplitude and frequency fluctuation; and
Fig. 4 is a timing chart showing variations in the rocking amplitude, frequency, and reclining angle of a backrest.

### BEST MODE FOR CARRYING OUT THE INVENTION

One embodiment of the present invention will now be discussed with reference to the drawings.

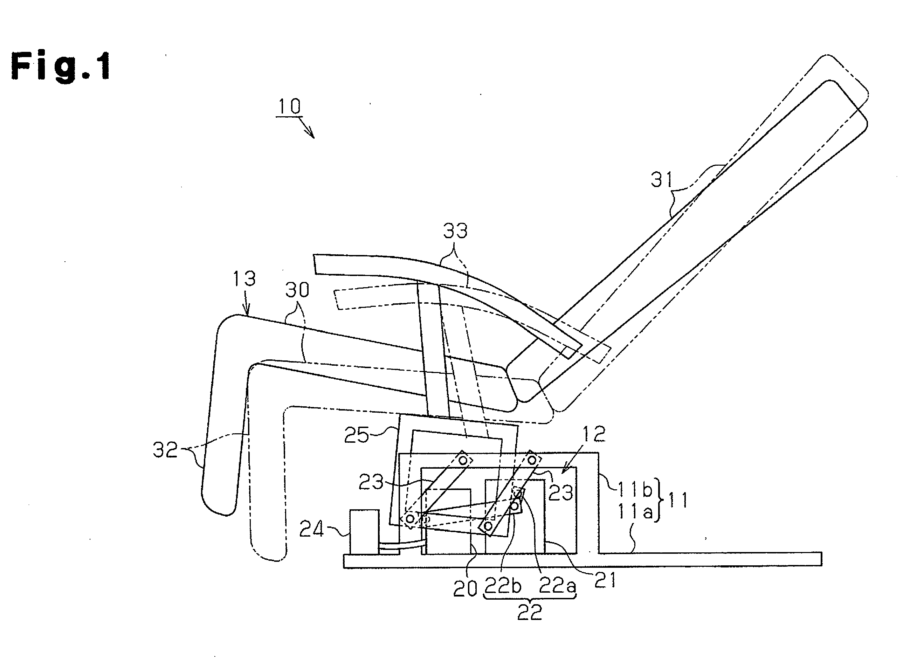

Fig. 1 is a schematic diagram showing a relaxation device 10 of the present embodiment. As shown in Fig. 1, the relaxation device 10 includes a rack 11 having a bottom portion 11a, which is arranged on a floor surface (not shown). A rocking driver 12, which serves as a rocking driving means, is arranged in the rack 11. The rocking driver 12 rocks a body support 13, which serves as a body supporting means.

The rocking driver 12 includes a motor 20, a speed reducer 21, a crank mechanism 22, and link members 23.

The motor 20 is arranged on an upper surface of the bottom portion 11 a of the rack 11. A controller 24, which serves as a control means (also referred to as a main control means) and a refreshment control means, is arranged on the upper surface of the bottom portion 11a. The controller 24 controls and drives the motor 20. The speed reducer 21 is arranged on the upper surface of the bottom portion 11a of the rack 11. The speed reducer 21 is connected to the motor 20 and outputs the drive force from the motor 20 at a reduced speed.

The crank mechanism 22, which includes two connection members 22a and 22b, converts the rotational drive of the speed reducer 21 to an arced motion of which diameter is extremely large. The connection member 22a has a basal end connected to an output shaft of the speed reducer 21 so as to be integrally rotatable with the output shaft. The connection member 22a has a distal end connected in a rotatable manner to a basal end of the connection member 22b. The connection member 22b has a distal end connected to a lower portion of a tetragonal frame-shaped base 25 fixed to the body support 13.

The rack 11 includes a support frame 11 b, extending upward from the bottom portion 11a. The two link members 23 have basal ends connected in a rotatable manner to an upper portion of the support frame 11b and spaced apart from each other by a predetermined distance. Further, the two link members 23 have distal ends connected in a rotatable manner to a lower portion of the base 25. Thus, the two link members 23 are each pivoted (swung) about its basal end and rock the body support 13, which is fixed to the base 25, in forward and rearward directions like a rocking chair when receiving drive force from the crank mechanism 22.

The chair-shaped body support 13 is fixed to the upper portion of the base 25. The body support 13 includes a seat 30, a backrest 31, a footrest 32, and armrests 33. The seat 30 is fixed to the base 25 so as to move integrally with the base 25. The inclinable backrest 31 is fixed to a rear part of the seat 30. The footrest 32 is fixed to a front part of the seat 30. The armrests 33 are arranged at the left and right sides of the seat 30.

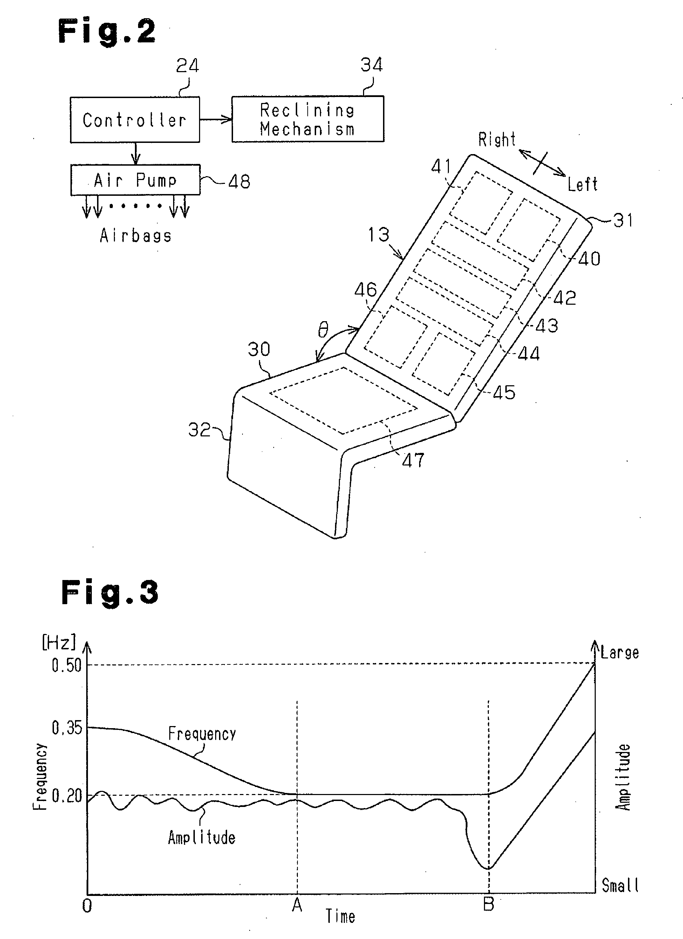

Referring to Fig. 2, the controller 24 controls a reclining mechanism 34, which inclines the backrest 31.

Airbags 40 and 41 are arranged in the upper left and right sides of the backrest 31 in correspondence with a user's left and right shoulders. Airbags 42, 43, and 44, which serve as back airbags, are arranged in the middle of the backrest 31 in correspondence with the user's back (upper back, middle back, and lower back). Airbags 45 and 46 are arranged in the lower left and right sides of the backrest 31 in correspondence with the user's left and right sides of the waist. An airbag 47 is arranged in the seat 30 in correspondence with the user's thighs. The airbags 40 to 47 are connected by connection hoses (not shown) to an air pump 48 arranged in a lower portion of the seat 30. The controller 24 controls and drives the air pump 48 to inflate and deflate each of the airbags 40 to 47.

The operation of the relaxation device 10 will now be discussed with reference to Figs. 1 to 4.

Various operation modes are set for the controller 24. The operation modes include a mode for generating relaxation rocking with the body support 13 by driving the rocking driver 12 and a mode that performs the relaxation rocking solely or in cooperation with the airbags 40 to 47 and the backrest 31. A preferred example further includes a refreshment function-added relaxation rocking mode.

The refreshment function-added relaxation rocking mode will now be discussed. In this operation mode, [relaxation rocking], which has a relaxing effect on the user, is first performed. The relaxation rocking is continued for a predetermined time, and then [relaxation rocking] is switched to [refreshment rocking]. This operation mode ends when the refreshment rocking is completed. The timing chart of Fig. 3 shows variations in the amplitude and frequency of the rocking of the body support 13 in the refreshment function-added relaxation rocking mode.

The refreshment function-added relaxation rocking mode will now be specifically described.

### [Relaxation Rocking]

The controller 24 first controls the reclining mechanism 34 and inclines the backrest 31 by a predetermined angle (e.g., 30 degrees) so that a reclining angle θ shifts from an upright state (e.g., reclining angle θ = 120 degrees) to a reclined state (e.g., reclining angle θ = 150 degrees). During the inclination, it is preferable that the movement speed of the backrest 31 be gradually reduced. Then, the controller 24 varies the rocking movement (frequency (cycle) and amplitude) of the body support 13 as time elapses. For example, the motor 20 is driven so that the rocking frequency of the body support 13 gradually decreases during the period from the starting of the rocking to time A, and so that a random fluctuation is produced in the amplitude of the body support 13. In the illustrated example, the initial value of the frequency is 0.35 Hz and gradually decreased by 0.05 Hz at a time until reaching 0.2 Hz (lower limit value of the frequency) at time A. Further, the controller 24 drives the air pump 48 from the starting of the rocking to time A so that the plurality of airbags 40 to 47, which are arranged in the backrest 31 and the seat 30, are inflated and deflated as required to perform massaging that rubs and stretches the user's body so as to relax the muscles.

During the period from time A to time B, the controller 24 controls the motor 20 so as to maintain the movement at time A, that is, maintain the rocking of the body support 13 at frequency 0.2 Hz, which is the lower limit value for the frequency in the present embodiment, while fluctuating the amplitude. This continues rocking and draws the user from a relaxed state to a sleep state. The rocking in the period from time A to time B may be referred to as sleep rocking. During the period from time A to time B, the controller 24 stops driving the air pump 48 and stops the operation of the airbags 40 to 47.

### [Refreshment Rocking]

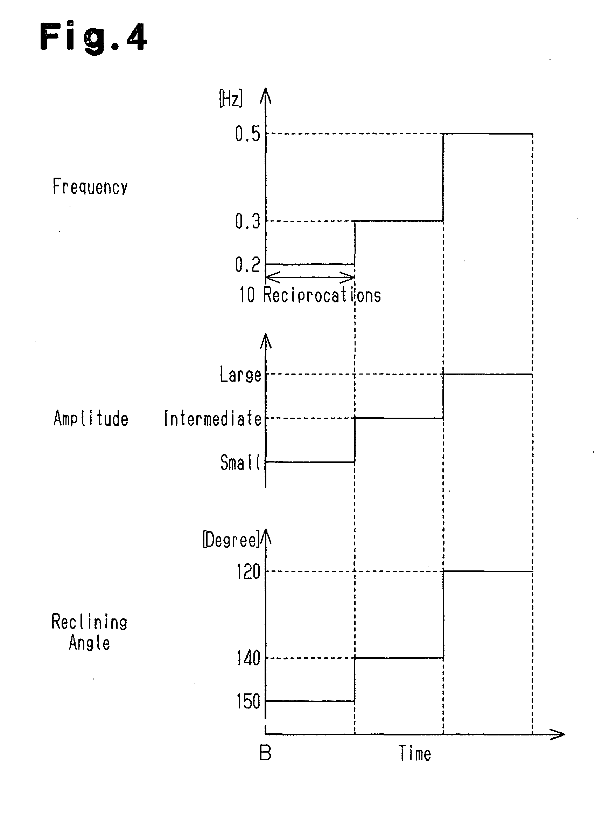

When a predetermined time elapses at time B, the controller 24 controls the motor 20 to vary the rocking frequency of the body support 13 so that the user is drawn from the sleep state (relaxed state) into an awake state. In the example illustrated in Fig. 4, the frequency is varied in a stepped manner so as to be increased from 0.2 Hz to 0.3 Hz, then continued at 0.3 Hz, and then varied from 0.3 Hz to 0.5 Hz. The controller 24 controls the timing for varying the frequency so that the frequency is varied after the body support 13 undergoes ten reciprocations, for example. The controller 24 controls the motor 20 and varies the level of the amplitude in synchronism with the timing for varying the frequency. In the example illustrated in Fig. 4, the amplitude increases in a stepped manner from "small" to "intermediate" and then from "intermediate" to "large". Further, the controller 24 controls the reclining mechanism 34 so that the backrest 31 is shifted in a stepped manner to the upright state (reclining angle θ = 120 degrees) in synchronism with the timings at which the frequency is varied. In this case, the controller 24 controls the reclining mechanism 34 so that the varying amount is increased in a stepped manner, such as from 150 degrees to 140 degrees and then from 140 degrees to 120 degrees. The movement speed of the backrest 31 may be controlled so that it gradually increases.

During the above sequences of the refreshment rocking, the controller 24 drives the air pump 48, which was not operating during the period from time A to time B and, for example, simultaneously inflates the left shoulder airbag 40 and the left waist airbag 45. The inflated state is maintained for five seconds, for example. Then, the airbag 40 and the airbag 45 are simultaneously deflated. Afterward, the right shoulder airbag 41 and the left waist airbag 46 are simultaneously inflated and maintained in the inflated state for five seconds, for example. In this manner, the controller 24 controls the air pump 48 by repetitively turning the user using the time difference between the lifting of the user's left side and the lifting of the user's right side. After the airbag operation ends, the controller 24 controls the air pump 48 to supply air to the airbags 42, 43, and 44 thereby lifting and stretching the user's back.

The controller 24 generates a control signal that controls parts such as the rocking driver 12 (motor 20), the reclining mechanism 34, and the air pump 48. In Fig. 3, the control signal that controls each part from the starting of the rocking to time A may be referred to as a first relaxation control signal. The control signal that controls each part during the period from time A to time B may be referred to as a second relaxation control signal. The control signal that controls each part after time B may be referred to as a refreshment control signal or an arousal control signal. The refreshment control means includes a circuit for generating the refreshment control signal.

The present embodiment has the advantages described below.
(1) Subsequent to the relaxation rocking, the controller 24 stops the fluctuation and controls the rocking driver 12 so as to perform the refreshment rocking that increases both the rocking frequency and amplitude of the body support to refresh the user who has been in a relaxed state. As a result, the rocking per unit time and the rocking width of the rocking movement are both increased. This produces a refreshing effect that draws the user from a relaxed state into an awake state. When the rocking frequency and amplitude are both increased, the refreshing effect on the user that draws the user from a relaxed state into an awake state is further effective. Further, by synchronously increasing the frequency and amplitude after a predetermined time elapses from when the rocking starts (e.g., at time B), the refreshing effect on the user for drawing the user from a relaxed state into an awake state is further effective.
(2) The controller 24 controls the motor 20 (rocking driver 12) so as to gradually decrease the frequency and fluctuate the amplitude during the relaxation rocking (the period from the start of the rocking to time A). This decreases the rocking frequency so as to assist the user's breathing and fluctuates the rocking amplitude thereby producing a relaxing effect that draws the user from an awake state into a relaxed state.
(3) During the relaxation rocking, the controller 24 varies the frequency within the range of 0.2 Hz to 0.4 Hz. This results in the frequency of the relaxation rocking being 0.2 Hz to 0.4 Hz, which is close to the breathing frequency when the user (person) is relaxed, and thus draws the user into a relaxed state. Further, the controller 24 controls the motor 20 (rocking driver 12) so that the rocking frequency and amplitude for the refreshment rocking are both greater than the frequency and amplitude for relaxation rocking. In the example of Fig. 3, the frequency range for the relaxation rocking is 0.2 Hz to 0.4 Hz, and the frequency range for the refreshment rocking is 0.2 Hz to 0.5 Hz. For example, by performing the refreshment rocking with a frequency that is greater than the breathing frequency when the user is relaxed, the refreshing effect on the user that draws the user into an awake state is further effective.
(4) The controller 24 controls the rocking driver so that the varying rate of the rocking frequency for the refreshment rocking is greater than the varying rate of the rocking frequency for the relaxation rocking. For example, in the illustrated example, the gradient of the frequency curve from time B is greater than the gradient of the frequency curve from time 0 to time B. In this manner, the varying rate of the rocking frequency during the refreshment rocking is greater than that during the relaxation rocking. This provides the user with a relatively strong stimulus during the refreshment rocking and has a further effective refreshing effect on the user.
(5) The body support 13 has the backrest 31 fixed in an inclinable manner to the rear part of the seat 30, on which the user may sit. The controller 24 inclines the backrest 31 to a reclined state during the relaxation rocking and raises the backrest 31 upright from the reclined state during the refreshment state. That is, by raising the backrest 31 upright, the user's upper body is also raised. This has a further effective refreshing effect on the user. Further, the user's body may be held in a state close to a state in which the user is lying down. This provides a relaxing effect.
(6) When raising the backrest 31 upright during the refreshment rocking, the controller 24 performs the raising in a stepped manner. This raises the user's upper body in a stepped manner. Thus, sudden changes in the user's blood pressure or the like are suppressed, and the burden on the user is suppressed.
(7) The controller 24 inclines the backrest 31 in synchronism with the timing for varying the frequency during the refreshment rocking. This has a further effective relaxing and refreshing effects on the user.
(8) The airbags 40 and 41 and the airbags 45 and 46, which are inflated and deflated when air from the air pump 48 is supplied or released, are arranged in pairs at the left side and right side of the backrest 31. During the refreshment rocking, the controller 24 inflates and deflates the left airbags 40 and 45 and the right airbags 41 and 46 with a time difference in between to stretch the user. This turns, or stretches, the user's right body half (upper right body half) and left body half (upper right body half) with a time difference and has a further effective refreshing effect on the user.
(9) The airbags 42, 43, and 44, which press the user's back when air from the air pump 48 is supplied or released, are arranged in the backrest 31. During the refreshment rocking, the controller 24 inflates and deflates the airbags 42, 43, and 44. This stretches mainly the user's back and has a further effective refreshing effect on the user.
(10) The controller 24 controls the air pump 48 to inflate and deflate the airbags 40 to 47 during the relaxation rocking in the period from the starting of the rocking to time A. Further, the controller 24 stops the inflation and deflation of the airbags 40 to 47 in a predetermined period (the period from time A to time B) during the relaxation rocking when sleep rocking is performed in which the rocking frequency is kept constant at the lower limit value and the amplitude is fluctuated. That is, by keeping the frequency constant at the lower limit value and fluctuating the amplitude while stopping operation of the air pump 48 to increase quietness, the user is drawn into a sleep state by a movement that mainly fluctuates in amplitude.

The embodiment of the present invention may be modified as described below.

In the above-discussed embodiment, the relaxation rocking is performed by fluctuating only the amplitude. However, the present invention is not limited in such a manner. For example, only the frequency may be fluctuated. Alternatively, the amplitude and the frequency may both be fluctuated.

In the above-discussed embodiment, the controller 24 controls the motor 20 so that the frequency for rocking the body support 13 (base 25) is 0.35 Hz to 0.2 Hz during the relaxation rocking. However, the motor 20 may be controlled to be in another frequency range that is less than 1 Hz.

In the above-discussed embodiment, the controller 24 controls the motor 20 so that the frequency for rocking the body support 13 (base 25) is 0.2 Hz to 0.5 Hz during the refreshment rocking. However, the motor 20 may be controlled to be in another frequency range that is less than 1 Hz.

In the above-discussed embodiment, the controller 24 controls the motor 20 so that the rocking amplitude and frequency are synchronously increased after a predetermined time elapses (for example, subsequent to time B). However, the amplitude and frequency need not be synchronized. Further, both of the amplitude and frequency do not have to be increased, and only one of the amplitude and frequency may be increased.

In the above-discussed embodiment, the backrest 31 is inclined during the relaxation rocking and the refreshment rocking but does not have to be inclined.

In the above-discussed embodiment, the timing for shifting the backrest 31 from a reclined state to an upright state is synchronized with the frequency varying timing but does not have to be synchronized. Further, the timing for shifting the backrest 31 from the reclined state to the upright state may be synchronized with an amplitude varying timing. Additionally, the timing for shifting the backrest 31 from the upright state to the reclined state may be synchronized with at least one of the amplitude and the frequency.

In the above-discussed embodiment, when raising the backrest during the refreshment rocking, the raising is performed in a stepped manner but does not have to be performed in such a manner.

Although not particularly mentioned, in the above-discussed embodiment, the controller 24 may control the reclining mechanism 34 so that the backrest 31 is inclined in a stepped manner when shifting to a reclined state during the relaxation rocking. Such a structure would lower the user's upper body half in a stepped manner. This would suppress sudden changes in the user's blood pressure or the like and suppress the burden on the user.

In the above-discussed embodiment, the movement speed of the backrest 31 is gradually decreased or increased. However, the present invention is not limited in such a manner and the speed may be constant. Further, the movement speed of the backrest 31 may be higher subsequent to time B (after the relaxation rocking ends) than during the relaxation rocking. This provides the user with a relatively strong stimulus during the refreshment rocking and has a further effective refreshing effect on the user.

In the above-discussed embodiment, the backrest 31 is inclinable but is not limited in such a manner and may be non-inclinable.

In the above-discussed embodiment, the airbags 40 and 45 and the airbags 41 and 46 are alternately inflated and deflated to produce a turning movement. However, the present invention is not limited in such a manner. For example, the airbag 47 arranged in the seat 30 and the back airbags 42, 43, and 44 may be arranged in pairs at the left side and right side. Further, the turning movement does not have to be performed.

In the above-discussed embodiment, a stretching movement that stretches the user's back is produced to lift and stretch the user's back. However, the stretching movement does not have to be produced.

In the above-discussed embodiment, the operation of the air pump 48 is stopped during the period between time A and time B. However, the present invention is not limited in such a manner, and the air pump 48 may continue to operate during the period from time A to time B.

In the above-discussed embodiment, after the turning movement produced by the airbags 40, 41, 45, and 46, the back airbags 42, 43, and 44 are inflated and deflated. However, the present invention is not limited in such a manner. For example, the inflation and deflation order may be reversed.

In the above-discussed embodiment, the body support 13 (seat 30 and backrest 31) includes the airbags 40 to 47 but does not have to.

In the above-discussed embodiment, the controller 24 switches control of each part at time A and time B. However, the present invention is not limited in such a manner. For example, a switch allowing the user to switch the control of each part by the controller 24 may be used to perform switching at a timing that is desirable for the user.

In the above-discussed embodiment, the body support 13 is chair-shaped. However, the present invention is not limited in such a manner. For example, the body support 13 may be bed-shaped or have any other shape.

In the above-discussed embodiment, the body support 13 is rocked in forward and rearward directions but is not limited in such a manner and may be rocked in sideward directions.

Although not particularly mentioned, in the above-discussed embodiment, music may be played and vibrations may be applied in correspondence with the relaxation rocking or refreshment rocking.

## Claims

1. A relaxation device (10) comprising:
a body supporting means (13) for supporting a user's body;
a rocking driving means (12) for rocking the body supporting means (13); and
a control means (24) for controlling the rocking driving means (12);
wherein the control means (24) controls the rocking driving means (12) to perform relaxation rocking that rocks the body supporting means (13) at a frequency in a range of 1 Hz or less and fluctuates at least either one of frequency and amplitude of the rocking; and
the control means (24) includes a refreshment control means that controls the rocking driving means (12) to perform, subsequent to the relaxation rocking, refreshment rocking that stops the fluctuation and increases at least either one of the rocking frequency and amplitude of the body supporting means (13) thereby refreshing the user who has been in a relaxed state, **characterized in that**
the body support means (13) is formed by a backrest (31) fixed in an inclinable manner to a rear part of a seat (30) on which the user seats;
the control means (24) inclines the backrest (31) to a reclined state during the relaxation rocking; and the refreshment control means raises the backrest (31) from the reclined state,
wherein at least either one of the seat (30) and the backrest (31) includes left and right airbags (40, 41, 45, 46) that are inflated and deflated when air from an air pump is supplied and released; and the refreshment control means controls the air pump so as to inflate and deflate the left airbag and the right airbag with a time difference during the refreshment rocking to turn the user.

2. The relaxation device (10) according to claim 1, being **characterized in that** the refreshment control means controls the rocking driving means (12) so as to increase both of the rocking frequency and amplitude.

3. The relaxation device (10) according to claim 2, being **characterized in that** the refreshment control means controls the rocking driving means (12) so as to synchronously increase both of the rocking frequency and amplitude.

4. The relaxation device (10) according to claim 1, being **characterized in that** the control means controls the rocking driving means so as to gradually decrease the frequency and fluctuate the amplitude during the relaxation rocking.

5. The relaxation device (10) according to claim 1, being **characterized in that**:
the control means (24) varies the frequency of the relaxation rocking in a range of 0.2 Hz to 0.4 Hz; and
the range of at least either one of the frequency and amplitude of the refreshment rocking performed by the refreshment control means is greater than that of the relaxation rocking.

6. The relaxation device (10) according to claim 1, being **characterized in that**:
the frequency of the refreshment rocking performed by the refreshment control means is varied at a rate that is greater than the rate at which the frequency of the relaxation rocking is varied.

7. The relaxation device (10) according to claim 1, being **characterized in that**:
the refreshment control means raises the backrest (31) in a stepped manner.

8. The relaxation device (10) according to claim 1, being **characterized in that**:
the control means (24) inclines the backrest (31) in a stepped manner to the reclined state during the relaxation rocking.

9. The relaxation device (10) according to claim 1, being **characterized in that**:
the control means (24) inclines the backrest (31) synchronously with the varying of either one of the frequency and the amplitude.

10. The relaxation device (10) according to claim 1, being **characterized in that**:
the refreshment control means inclines the backrest (31) so that the speed at which the backrest moves is higher during the refreshment rocking than the speed at which the backrest (31) moves during the relaxation rocking.

11. The relaxation device (10) according to claim 1, being **characterized in that**:
the backrest (31) includes a back airbag that presses the user's back by supplying and releasing the air from the air pump; and
the refreshment control means controls the air pump so as to inflate and deflate the back airbag during the refreshment rocking.

12. The relaxation device (10) according to claim 1 or 11, being **characterized in that**:
the control means (24) also controls the air pump to inflate and deflate the airbags during the relaxation rocking; and
the control means (24) keeps the rocking frequency at a lower limit value, fluctuates the amplitude, and stops inflating and deflating the airbags when sleep rocking is performed during the relaxation rocking.

## Patentansprüche

1. Entspannungsvorrichtung (10), umfassend:
ein Körperauflagemittel (13) zum Stützen eines Körpers eines Benutzers;
ein Schaukelantriebsmittel (12) zum Schaukeln des Körperauflagemittels (13); und
ein Steuermittel (24) zum Steuern des Schaukelantriebsmittels (12);
wobei das Steuermittel (24) das Schaukelantriebsmittel (12) steuert, um ein Entspannungsschaukeln auszuführen, wobei das Körperauflagemittel (13) bei einer Frequenz in einem Bereich von 1 Hz oder weniger schaukelt und zumindest eines von einer Frequenz und Amplitude des Schaukelns fluktuiert; und
das Steuermittel (24) ein Erfrischungssteuermittel enthält, das das Schaukelantriebsmittel (12) steuert, um nach dem Entspannungsschaukeln ein Erfrischungsschaukeln durchzuführen, das die Fluktuation stoppt und zumindest eines von der Schaukelfrequenz und -amplitude des Körperauflagemittels (13) erhöht, wodurch der Benutzer, der in einem entspannten Zustand war, erfrischt wird, **dadurch gekennzeichnet, dass**
das Körperauflagemittel (13) durch eine Rückenlehne (31) gebildet ist, die schrägstellbar an einem hinteren Teil eines Sitzes (30) befestigt ist, auf dem der Benutzer sitzt;
das Steuermittel (24) die Rückenlehne (31) während des Entspannungsschaukelns in einen zurückgelehnten Zustand schrägstellt; und das Erfrischungssteuermittel die Rückenlehne (31) aus dem zurückgelehnten Zustand anhebt,
wobei zumindest eines von dem Sitz (30) und der Rückenlehne (31) ein linkes und rechtes Luftpolster (40, 41, 45, 46) enthält, die aufgeblasen und entleert werden, wenn Luft von einer Luftpumpe zugeleitet und abgeleitet wird; und das Erfrischungssteuermittel die Luftpumpe so steuert, dass das linke Luftpolster und das rechte Luftpolster mit einer Zeitdifferenz während des Erfrischungsschaukelns aufgeblasen und entleert werden, um den Benutzer zu drehen.

2. Entspannungsvorrichtung (10) nach Anspruch 1, **dadurch gekennzeichnet, dass** das Erfrischungssteuermittel das Schaukelantriebsmittel (12) so steuert, dass die Schaukelfrequenz und -amplitude erhöht werden.

3. Entspannungsvorrichtung (10) nach Anspruch 2, **dadurch gekennzeichnet, dass** das Erfrischungssteuermittel das Schaukelantriebsmittel (12) so steuert, dass die Schaukelfrequenz und -amplitude gleichzeitig erhöht werden.

4. Entspannungsvorrichtung (10) nach Anspruch 1, **dadurch gekennzeichnet, dass** das Steuermittel das Schaukelantriebsmittel so steuert, dass während des Entspannungsschaukelns die Frequenz allmählich verringert wird und die Amplitude fluktuiert.

5. Entspannungsvorrichtung (10) nach Anspruch 1, **dadurch gekennzeichnet, dass**:
das Steuermittel (24) die Frequenz des Entspannungsschaukelns in einem Bereich von 0,2 Hz bis 0,4 Hz variiert; und
der Bereich von zumindest einer der Frequenz oder Amplitude des Erfrischungsschaukelns, das vom Erfrischungssteuermittel ausgeführt wird, größer ist als jener des Entspannungsschaukelns.

6. Entspannungsvorrichtung (10) nach Anspruch 1, **dadurch gekennzeichnet, dass**:
die Frequenz des Erfrischungsschaukelns, das vom Erfrischungssteuermittel ausgeführt wird, bei einer Rate variiert wird, die größer als die Rate ist, bei der die Frequenz des Entspannungsschaukelns variiert wird.

7. Entspannungsvorrichtung (10) nach Anspruch 1, **dadurch gekennzeichnet, dass**:
das Erfrischungssteuermittel die Rückenlehne (31) stufenweise anhebt.

8. Entspannungsvorrichtung (10) nach Anspruch 1, **dadurch gekennzeichnet, dass**:
das Steuermittel (24) die Rückenlehne (31) während des Entspannungsschaukelns stufenweise in den zurückgelehnten Zustand schrägstellt.

9. Entspannungsvorrichtung (10) nach Anspruch 1, **dadurch gekennzeichnet, dass**:
das Steuermittel (24) die Rückenlehne (31) synchron mit dem Variieren entweder der Frequenz oder Amplitude schrägstellt.

10. Entspannungsvorrichtung (10) nach Anspruch 1, **dadurch gekennzeichnet, dass**:
das Erfrischungssteuermittel die Rückenlehne (31) so schrägstellt, dass die Geschwindigkeit, mit der sich die Rückenlehne während der Erfrischungssteuerung bewegt, höher ist als die Geschwindigkeit, mit der sich die Rückenlehne (31) während des Entspannungsschaukelns bewegt.

11. Entspannungsvorrichtung (10) nach Anspruch 1, **dadurch gekennzeichnet, dass**:
die Rückenlehne (31) ein hinteres Luftpolster enthält, das gegen den Rücken des Benutzers presst, indem die Luft aus der Luftpumpe zugeleitet und abgeleitet wird; und
das Erfrischungssteuermittel die Luftpumpe so steuert, dass das hintere Luftpolster während des Erfrischungsschaukelns aufgeblasen und entleert wird.

12. Entspannungsvorrichtung (10) nach Anspruch 1 oder 11, **dadurch gekennzeichnet, dass**:
das Steuermittel (24) auch die Luftpumpe steuert, um die Luftpolster während des Entspannungsschaukelns aufzublasen und zu entleeren; und
das Steuermittel (24) die Schaukelfrequenz bei einem unteren Grenzwert hält, die Amplitude fluktuiert und ein Aufblasen und Entleeren der Luftpolster stoppt, wenn ein Schlafschaukeln während des Entspannungsschaukelns ausgeführt wird.

## Revendications

1. Dispositif de relaxation (10) qui comprend :
un moyen de support du corps (13) destiné à supporter le corps d'un utilisateur ;
un moyen de balancement (12) destiné à balancer le moyen de support du corps (13) ; et
un moyen de commande (24) destiné à contrôler le moyen de balancement (12) ;
dans lequel le moyen de commande (24) contrôle le moyen de balancement (12) afin d'assurer un balancement de relaxation qui balance le moyen de support du corps (13) à une fréquence de 1 Hz ou moins, et fait fluctuer la fréquence ou l'amplitude du balancement ; et
le moyen de commande (24) comprend un moyen de commande de rafraîchissement qui contrôle le moyen de balancement (12) afin d'assurer, après le balancement de relaxation, un balancement de rafraîchissement qui arrête la fluctuation et augmente au moins l'une de la fréquence et de l'amplitude de balancement du moyen de support du corps (13), afin de rafraîchir l'utilisateur qui se trouve dans un état de relaxation, **caractérisé en ce que**
le moyen de support du corps (13) est formé d'un dossier (31) fixé de manière inclinable sur une partie arrière d'un siège (30) sur lequel est assis l'utilisateur ;
le moyen de commande (24) incline le dossier (31) pendant le balancement de relaxation ; et le moyen de commande de rafraîchissement remonte le dossier (31),
dans lequel au moins l'un du siège (30) et du dossier (31) comprend des airbags gauches et droits (40, 41, 45, 46) qui sont gonflés et dégonflés lorsque de l'air apporté par une pompe à air est fourni puis libéré ; et le moyen de commande de rafraîchissement contrôle la pompe à air de façon à gonfler et dégonfler l'airbag de gauche et l'airbag de droite avec un écart de temps pendant le balancement de rafraîchissement, de façon à faire tourner l'utilisateur.

2. Dispositif de relaxation (10) selon la revendication 1, **caractérisé en ce que** le moyen de commande de rafraîchissement contrôle le moyen de basculement (12) de façon à augmenter la fréquence et l'amplitude de basculement.

3. Dispositif de relaxation (10) selon la revendication 2, **caractérisé en ce que** le moyen de commande de rafraîchissement contrôle le moyen de basculement (12) de façon à augmenter de manière synchrone la fréquence et l'amplitude de basculement.

4. Dispositif de relaxation (10) selon la revendication 1, **caractérisé en ce que** le moyen de commande contrôle le moyen de basculement de façon à réduire progressivement la fréquence et à faire fluctuer l'amplitude pendant le basculement de relaxation.

5. Dispositif de relaxation (10) selon la revendication 1, **caractérisé en ce que** :
le moyen de commande (24) fait varier la fréquence du basculement de relaxation entre 0,2 et 0,4 Hz ; et
la plage d'au moins l'une de la fréquence et de l'amplitude du basculement de rafraîchissement effectué par le moyen de commande de rafraîchissement est supérieure à celle du basculement de relaxation.

6. Dispositif de relaxation (10) selon la revendication 1, **caractérisé en ce que** :
la fréquence du basculement de rafraîchissement effectué par le moyen de commande de rafraîchissement est variée à une vitesse supérieure à celle à laquelle la fréquence du basculement de relaxation est variée.

7. Dispositif de relaxation (10) selon la revendication 1, **caractérisé en ce que** :
le moyen de commande de rafraîchissement remonte le dossier (31) de manière progressive.

8. Dispositif de relaxation (10) selon la revendication 1, **caractérisé en ce que** :
le moyen de commande (24) incline le dossier (31) de manière progressive pendant le basculement de relaxation.

9. Dispositif de relaxation (10) selon la revendication 1, **caractérisé en ce que** :
le moyen de commande (24) incline le dossier (31) de manière synchrone avec la variation de l'une de la fréquence et de l'amplitude.

10. Dispositif de relaxation (10) selon la revendication 1, **caractérisé en ce que** :
le moyen de commande de rafraîchissement incline le dossier (31) de sorte que la vitesse à laquelle le dossier se déplace soit plus élevée pendant le basculement de rafraîchissement que la vitesse à laquelle le dossier (31) se déplace pendant le basculement de relaxation.

11. Dispositif de relaxation (10) selon la revendication 1, **caractérisé en ce que** :
le dossier (31) comprend un airbag arrière qui appuie sur le dos de l'utilisateur en fournissant et en libérant de l'air depuis la pompe à air ; et
le moyen de commande de rafraîchissement contrôle la pompe à air de façon à gonfler et à dégonfler l'airbag arrière pendant le basculement de rafraîchissement.

12. Dispositif de relaxation (10) selon la revendication 1 ou 11, **caractérisé en ce que** :
le moyen de commande (24) contrôle également la pompe à air de façon à gonfler et dégonfler les airbags pendant le basculement de relaxation ; et
le moyen de commande (24) maintient la fréquence de basculement à une valeur limite inférieure, fait fluctuer l'amplitude, et arrête de gonfler et de dégonfler les airbags lorsqu'un basculement de sommeil est effectué pendant le basculement de relaxation.
